Europäisches Patentamt

(19) European Patent Office

Office européen des brevets

(11) Publication number: **0 040 457**
**B1**

(12) **EUROPEAN PATENT SPECIFICATION**

(45) Date of publication of patent specification: **24.04.85**

(21) Application number: **81300287.0**

(22) Date of filing: **22.01.81**

(51) Int. Cl.⁴: **A 61 K 9/00,** A 61 K 9/22, A 61 M 31/00

(54) Agent dispenser with microporous releasing diffusor.

(30) Priority: **28.04.80 US 144210**

(43) Date of publication of application:
**25.11.81 Bulletin 81/47**

(45) Publication of the grant of the patent:
**24.04.85 Bulletin 85/17**

(84) Designated Contracting States:
**AT BE CH DE FR GB IT LI NL SE**

(56) References cited:
**FR-A-2 121 830**
**FR-A-2 371 224**
**FR-A-2 386 316**

(73) Proprietor: **ALZA CORPORATION**
**950 Page Mill Road**
**Palo Alto, California 94304 (US)**

(72) Inventor: **Theeuwes, Felix**
**1643 Fallen Leaf Lane**
**Los Altos California (US)**

(74) Representative: Evans, David Charles et al
**F.J. CLEVELAND & COMPANY 40-43, Chancery**
**Lane**
**London, WC2A 1JQ (GB)**

Courier Press, Leamington Spa, England.

# Description

## Technical field

This invention pertains to a dispenser comprising a microporous diffusor for delivering a beneficial agent to a fluid environment of use.

## Background art

Dispensers for delivering an active agent to a water containing environment of use are known. For example, in United States Patent Nos. 3,845,770 and 3,916,899 issued to inventors Felix Theeuwes and Takeru Higuchi, osmotic dispensers are disclosed for delivering a beneficial agent at a controlled rate to a fluid environment of use. The dispensers disclosed in these patents comprise a semipermeable wall that surround a compartment containing the agent. The wall is permeable to an external fluid, substantially impermeable to agent, and there is a passageway through the wall for delivering the agent from the dispenser. These dispensers release the agent by fluid being imbibed through the wall into the compartment, at a rate determined by the permeability of the wall and the osmotic pressure gradient across the wall, to produce a solution of the agent, that is dispensed through a passageway from the dispenser. In United States Patent No. 4,111,202, issued to Felix Theeuwes, an osmotic dispenser is disclosed comprising a semipermeable wall that surrounds a first and second compartment. The first compartment contains an active agent and the second compartment contains an osmotically effective solute. A passageway through the semipermeable wall connects the exterior of the system with the first compartment. The dispenser releases agent by fluid being imbibed into the first compartment to prepare a solution containing agent and by fluid being imbibed into the second compartment causing it to increase in volume expand into and decrease the volume of the first compartment, whereby the agent is delivered through the passageway from the dispenser.

According to the present invention there is provided a dispenser for the controlled delivery of an active agent into a fluid environment, the dispenser comprising an exterior wall surrounding and forming an internal compartment, a flexible partition in the compartment separating the compartment into a first space and a second space, an active agent in the first space and a swellable lightly cross-linked polymer or an osmotically effective solute in the second compartment, the dispenser characterized in that (a) the exterior wall is formed of a microporous polymer when the second space contains a swellable cross-linked polymer, or (b) the exterior wall is formed of a microporous and a semipermeable polymer with the semipermeable polymer surrounding a part of the second space containing a swellable cross-linked polymer or an osmotically effective solute, and in that the external wall totally encloses the internal compartment and the microporous polymer in (a) or (b) permits the passage of active agent from the dispenser to the environment.

The dispenser operates as follows when placed in a water-containing environment. Water from the environment enters the first and second spaces. In dispenser (1) a solution of active agent is formed in the first space which solution diffuses through the microporous wall to the exterior of the dispenser, while the second space of dispenser (1), the swellable polymer absorbs water causing it to expand against the partition, thereby causing the partition to move into the first space and urge the solution of the active agent in the first space towards the microporous wall for release from the dispenser. In dispenser (2), the semipermeable wall permits water to enter the second space and form a solution of osmotically effective solute, that fills the space and pushes against the partition, thereby urging the partition to move into the first space causing the solution of the active agent to move towards the microporous wall for release from the dispenser. In dispenser (2) when the second space contains a swellable polymer, the dispenser operates as described above.

## Brief description of the drawings

In the drawings:

Figure 1 is a view of a dispenser designed and shaped for administering orally a drug to a warm-blooded animal;

Figure 2a is an opened view of the dispenser of Figure 1, which Figure 2a illustrates an external microporous wall and the internal structure of the dispenser consisting essentially of two spaces separated by a partition;

Figure 2b is similar to Figure 2a, illustrating a dispenser with an external microporous semipermeable wall;

Figure 3 illustrates, in opened section, a dispenser shaped and dimensioned for dispensing a drug in a body passageway such as the vagina, or the ano-rectal passageway.

## Detailed description of embodiment of invention

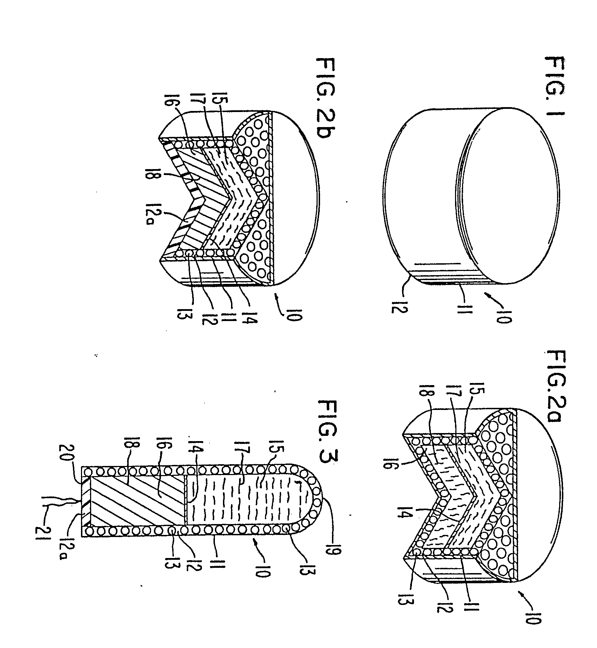

In Figure 1, a dispenser 10 is seen comprising a body 11 that is shaped and adapted for easy use as a tablet.

In Figure 2a dispenser 10 of Figure 1 is seen in opened-section with a part of its outer layer removed for elucidating the total structure of dispenser 10. In Figure 2a, dispenser 10 comprises a body 11 having an exterior wall 12 that surrounds an internal space having a partition 14 that separates the space into a first space 15 and a second space 16. Space 16 contains a swellable polymer 18, that on swelling in the presence of water exerts pressure on partition 14 causing it to move and occupy volume in space 15. The actions of partition 14 and polymer 18 combine to decrease the volume of space 15, thereby functioning to maintain active agent 17 in a saturated state in space 15, especially during the time dispenser 10 is in operation in a prechosen environment of use.

**0 040 457**

In Figure 2a, wall 12 of dispenser 10 is formed of a microporous polymeric material containing a plurality of microscopic-sized interconnected pores or voids. The pores, illustrated as circles 13 for discussion herein, can be continuous with openings on both sides of wall 12, the pores can be interconnected through tortuous paths of regular and irregular shapes, including curved, curved-linear, randomly oriented continuous paths, hindered connected paths and pores, and other paths and pores discernible by microscopic examination. Generally, materials possessing from 5 to 95% pores, more preferably a void space of 30% to 90%, and having a pore size of 100 angstroms to 200 microns can be used for making wall 12. The pores and connecting intra-wall paths can be preformed in the polymer, which microporous polymer is then manufactured as wall 12 of dispenser 10. In another, and presently preferred embodiment, wall 12 contains a multiplicity of pore-formers, not shown, that are dissolved or leached from wall 12, which is integrally manufactured as dispenser 10. In this embodiment, the pore-formers are removed when dispenser 10 is in the environment of use, thereby forming microporous wall 12 in the environment.

The microporous paths of wall 12 are prefilled or filled in the environment of use with a diffusive medium permeable to the passage of agent 17. The medium is generally non-toxic and it does not adversely effect the dispenser, the wall, the agent and the environment. In one embodiment, the medium is a liquid phase comprised of a solution, a colloidal medium, or a sol, the medium can be polar, semi-polar or non-polar, or it can be a liquid present in the environment of use, including water, biological fluids, saline, and buffers.

Partition 14 of dispenser 10 consists, in one embodiment, of a film made of a semipermeable polymer that is essentially impermeable to the passage of agent, osmotic solute and polymer and is permeable to the passage of fluid that enters dispenser 10; and, in another embodiment partition 14 is made of a film impermeable to agent, solutes, polymers and fluid. Partition 14 is suitably joined to wall 12 during manufacture of dispenser 10, and in a presently preferred embodiment it can contain a plasticizer that imparts flexibility and expandibility to partition 14. In operation, when compartment 16 contains polymer 18, the polymer 18 absorbs fluid that enters compartment 16 causing 18 to swell, expand and fill compartment 16, and also, swell and expand against partition 14, causing it to move and occupy the space of compartment 15. This action correspondingly reduces the amount of space available for agent 17, and this continual decrease in space substantially keeps agent 17 in a substantially saturated phase.

In Figure 2b, another dispenser 10 is provided according to the mode and the manner of invention. Dispenser 10 of Figure 2b is similar to system 10 of Figure 2a, with dispenser 10 of Figure 2b embracing other structural embodiments. The embodiments of Figure 2b include wall 12 having at least one surface 12a formed of a semipermeable polymer. When wall 12a is formed of a semipermeable polymer, space 16 contains a member selected from the group consisting essentially of an osmotically effective solute or a swellable polymer 18. When space 16 houses the osmotic solute or the swellable polymer, partition 14 is formed of a semipermeable polymer, or an impermeable polymer. When space 16 contains an osmotic solute, in operation it imbibes fluid through semipermeable wall 12a in a tendency towards osmotic equilibrium, to dissolve the solute and form a solution that fills space 16, apply pressure against partition 14, urging it to move into space 15 and decrease its volume, thereby keeping the beneficial agent present at the microporous wall 12. When space 16 contains a swellable polymer, it absorbs fluid, expands, but does not dissolve in fluid that enters space 16. The expanding polymer pushes against partition 14 causing it to move into space 15, thereby keeping agent 17 in a saturated state at the release rate wall.

Figure 3 shows a dispenser 10 designed, shaped, sized and styled for easy placement and comfortable retention in a body passageway, such as the vagina, or the ano-rectal passageways. Dispenser 10 has an elongated, cylindrical, self-sustaining shape with a pointed lead end 19, a trailing end 20, and it is equipped with manually controlled cords 21 for easily removing dispenser 10 from a body passageway. Dispenser 10 of Figure 3 is structurally identical with dispenser 10 of Figure 1, 2a and 2b, and it operates in a like manner, with element 16 expanding for continually occupying area and void space in element 15 created by agent 17 diffusing through micropores 13. Dispenser 10 of Figure 3 contains a drug designed for release and absorption by the vaginal or the rectal mucosa.

The microporous materials that can be used for manufacturing dispenser 10 are known materials. The microporous materials can be made, and then manufactured into a system, by etched nucelar tracking, by cooling a solution of a flowable polymer below its freezing point whereby solvent evaporates from the solution in the form of crystals dispersed in the polymer, and then curing the polymer followed by removing the solvent crystals, by cold or hot stretching of a polymer at low or high temperatures until pores are formed, by leaching from a polymer a soluble pore forming component by use of an appropriate solvent, by ion exchange reactions consisting of exchanging a large space occupying ion with a smaller ion, by polyelectrolytic processes, and by dissolving or leaching a pore former from the wall of a dispenser in operation in the environment of use. Processes for preparing microporous materials are described in *Synthetic Polymer Membranes*, by R. E. Kesting, Chapters 4 and 5, 1971 published by McGraw Hill, Inc; *Chemical Reviews, Ultrafiltration*, Vol. 18, pages 373 to 455, 1934; *Polymer Eng. and Sci.*, Vol. 11, No. 4, pages 284 to 288, 1971; *J. Appl. Poly. Sci.*, Vol. 15, pages 811 to 829, 1971; and in United States Patent Nos. 3,565,259; 3,615,024; 3,751,536; 3,801,692; 3,852,224; and 3,849,528.

Materials useful for making the microporous wall 12 include polycarbonates comprised of linear polyesters of carbonic acid in which carbonate groups recur in the polymer chain, microporous materials prepared by the phosgenation of a dihydroxyl aromatic such as a bisphenol A, microporous poly(vinylchloride), microporous polyamides such as polyhexamethylene adipamide, microporous modacrylic copolymers including those formed from poly(vinylchloride) 60% and acrylonitrite, microporous styrene-acrylic copolymers, porous polysulfones characterized by diphenylene sulfone groups in a linear chain thereof, halogenated poly(vinylidene), polychloroethers, acetal polymers, polyesters prepared by esterification of a dicarboxylic acid or anhydrid with an alkylene polyol, poly(alkylenesulfides), phenolic polyesters, microporous poly(saccharides), microporous poly(saccharides) having substituted anhydrogluclose units exhibiting a decreased permeability to the passage of water and biological fluids, asymmetric porous polymers, cross-linked microporous olefin polymers, hydrophobic or hydrophilic microporous homopolymers, copolymers having a reduced bulk density, and materials described in United States Patent Nos. 3,595,752; 3,643,178; 3,654,066; 3,709,774; 3,718,532; 3,803,061; 3,852,224; 3,852,388; and 3,853,601, in British Patent No. 1,126,849, and in *Chem. Abst.*, Vol. 71 427F, 22573F, 1969.

The pore-formers useful for forming microporous wall 12 in the environment of use include solids and poreforming liquids. In the latter expression, the term for this invention generically embraces semi-solids and viscous fluids. The pore-formers can be inorganic or organic and the wall forming polymer usually contains from 5 to 95% by weight. The term pore-formers that can be dissolved, leached, or extracted without causing physical or chemical changes in the polymer. The pore-forming solids have a size of about 100 angstroms to 200 microns, and they include alkali metal salts such as lithium carbonate, sodium chloride, sodium bromide, sodium carbonate, potassium chloride, potassium sulfate, potassium phosphate, sodium benzoate, sodium acetate, sodium citrate, potassium nitrite and the like. The alkaline earth metal salts such as calcium phosphate, calcium nitrate, calcium chloride, and the like. The transition metal salts such as ferric chloride, ferrous sulfate, zinc sulfate, cupric chloride, manganese fluoride, manganese fluorosilicate, and the like. Organic compounds such as polysaccharides including pentoses, hexoses, disaccharides, sugars, sucrose, glucose, fructose, mannitol, mannose, galactose, aldohexose, altrose, talose, sorbitol, and the like, carboxy-polymethylene, Carbowax® compounds, polysorbate, and the like. The pore-formers are non-toxic and on their removal from the wall, channels or paths are formed through wall 12, that fill with fluid. The paths become a means, or diffusional path for diffusion of active agent, or drug from the system. The pores extend from inside wall 12 to the outside of wall 12 for effective release of active agent or drug to the exterior of dispenser 10.

Additional microporous materials for forming wall 12 include microporous poly(urethanes), cross-linked chain-extended microporous poly(urethanes), microporous poly(urethanes) in United States Patent No. 3,524,753, microporous poly(imides), microporous poly(benzimidazoles), regenerated microporous proteins, semi-solid cross-linked microporous poly(vinylpyrrolidone), microporous materials prepared by diffusion of multivalent cations into polyelectrolyte sols as in United States Patent No. 3,565,259, anisotropic permeable microporous materials of ionically associated polyelectrolytes, microporous polymers formed by the coprecipitation of a polycation and a polyanion as described in United States Patent Nos. 3,276,589; 3,541,006; 3,541,055; and 3,546,142, microporous derivatives of poly(styrene) such as microporous poly(sodium styrene-sulfonate) and microporous poly(vinyl benzyltrimethyl-ammonium chloride), the microporous materials disclosed in United States Patent No. 3,615,024 and United States Patent Nos. 3,646,178 and 3,852,224.

The selectively permeable polymers used for partition 14 and wall 12a, when a semipermeable polymer is used for their manufacture in dispenser 10, include, polymers permeable to fluid present in dispenser 10 and the environment, while remaining impermeable to solutes, active agents and drugs. Typical materials include semipermeable polymers, also known to the art as osmosis membranes. The semipermeable polymers include cellulose acylate, cellulose diacylate, cellulose triacylate, cellulose ethers and cellulose esters. Typical semipermeable polymers include cellulose acetate, cellulose acetate ethyl carbamate, and the like. Other semipermeable polymers include polyurethane, and selectively permeable polymers formed by the coprecipitation of a polyanion and a polycation, and semipermeable ion exchange polymers. Generally, semipermeable polymers useful for forming partition 14, or wall 12a, will have a fluid permeability of $10^{-5}$ to $10^{-1}$ (cc mil/cm$^2$ hr atm) expressed per atmosphere of hydrostatic or osmotic pressure difference across 14 or 12a at the temperature of use.

Exemplary polymers suitable for partition 14, when it is impermeable to fluid agents and solutes include, plasticized polyvinyl chloride, styrene-butadiene block copolymer, polyesterpolyethers, ethylene-propylene copolymer, segmented block polyurethane, chlorinated polyethylene, ethylene vinylchloride copolymer, and the like. The partition in both designs, can contain a plasticizer to increase its flexibility during use.

Exemplary plasticizers suitable for adding to partition 14 to impart flexibility and stretchability include cyclic and acyclic plasticizers. Typical plasticizers are those selected from the group consisting of phthalates, phosphates, citrates, adipates, tartrates, sebacates, succinates, glycolates, glycerolates, benzoates, myristates, sulfonamides, halogenated phenyls, glycols, diols, and polyols.

Exemplary plasticizers further include dialkyl phthalates, dicycloalkyl phthalates, diaryl phthalates and mixed alkyl-aryl phthalates as represented by dimethyl phthalate, dipropyl phthalate, di(2-ethylhexyl)-phthalate, diisopropyl phthalate, diamyl phthalate and dicapryl phthalate; alkyl and aryl phosphates such as tributyl phosphate, trioctyl phosphate, tricresyl phosphate, and triphenyl phosphate; alkyl citrate and citrates esters such as tributyl citrate, triethyl citrate, and acetyl triethyl citrate; alkyl adipates such as dioctyl adipate, diethyl adipate and di-(2-methoxyethyl)-adipate; dialkyl tartrates such as diethyl tartrates and dibutyl tartrate; alkyl sebacates such as diethyl sebacate, dipropyl sebacate and dinonyl sebacate; alkyl succinates such as diethyl succinate and dibutyl succinate; alkyl glycolates, alkyl glycerolates, glycol esters and glycerol esters such as glycerol diacetate, glycerol triacetate, glycerol monolactate diacetate, methyl phthalyl ethyl glycolate, butyl phthalyl butyl glycolate, ethylene glycol diacetate, triethylene glycol dibutyrate and triethylene glycol dipropionate. Other plasticizers include camphor, N-ethyl-(o- and p-toluene) sulfonamide, chlorinated biphenyl, benzophenone, N-cyclohexyl-p-toluene sulfonamide, substituted epoxides, poly(alkylene glycols), poly(alkylene diols), esters of alkylene glycols, and the like.

Suitable plasticizers can be selected for blending with partition 14 forming materials by selecting plasticizers that have a high degree of solvent power for the materials, are compatible with the materials over both the processing and use temperature ranges, exhibit permanance as seen by a strong tendency to remain in the plasticized partition and imparts flexibility to the partition. Procedures for selecting a plasticizer having the described characteristics are disclosed in the *Encyclopedia of Polymer Science and Technology*, Vol. 10, pages 228 to 306, 1979, published by John Wiley & Sons, Inc., New York. Also, a detailed description pertaining to the measurement of plasticizer properties, including solvent parameters and compatibility, the Hildebrand solubility parameter, the Flory-Huggins interaction parameter, and the cohesive-energy density, CED, parameter is disclosed in *Plasticization and Plasticizer Processes, Advances in Chemistry Series 48*, Chapter 1, pages 1 to 26, 1965, published by the American Chemical Society, Washington, D.C. The amount of plasticizer added generally is an amount sufficient to produce the desired film and it will vary according to the plasticizer and the materials. Usually about 0.001 part up to 25 parts, or higher, of the plasticizer can be used for 100 parts partition forming material with a presently preferred range of 0.1 part to 15 parts of plasticizer, or mixtures thereof for 100 parts of partition forming materials.

The swellable polymer that can be used as driving member 18 for expanding and enlarging space 16, and for pushing partition 14, as in Figures 2a and 2b, into agent space 15, or for swelling and expanding while correspondingly decreasing the agent containing space, are generally lightly cross-linked hydrophilic polymers. These polymers, on swelling, reduce the amount of space available for agent 17, and this continual decrease in space acts to substantially maintain agent 17 in a substantially saturated phase. The formulation and maintenance at the agent microporous wall boundary layer in system 10, at substantially the same rate and amount throughout the release period, produces for dispenser 10, a prolonged zero order rate.

Representative polymers are those that swell in the presence of fluid to a high degree without dissolution, are lightly cross-linked, usually exhibiting a 5 to 50 fold volume increase. Exemplary polymers are cross-linked hydrogels including poly(hydroxyalkylmethacrylates), poly-(acrylamide), poly(methacrylamide), poly-(N-vinyl-2-pyrrolidone), anionic and cationic hydrogels, polyelectrolyte complexes, a water-insoluble, water-swellable copolymer produced by forming a dispersion of finely divided copolymers of maleic anhydride with styrene, ethylene propylene, butylene or isobutylene cross-linked with from about 0.001 to about 0.5 moles of a polyunsaturated cross-linking agent per mole of maleic anhydride in the copolymer as disclosed in U.S. Patent No. 3,989,586, the water-swellable polymers of N-vinyl lactams as disclosed in U.S. Patent No. 3,992,562, semi-solid cross-linked poly(vinyl pyrrolidone), diester cross-linked poly-glucan hydrogels as described in U.S. Patent No. 4,002,173, the anionic hydrogels of heterocyclic N-vinyl monomers as disclosed in U.S. Patent No. 4,036,788, the ionogenic hydrophillic gels as described in *J. Biomedical Mater. Res.*, Vol. 7, pages 123 to 126, 1973, and the like.

The osmotically effective compound that can be used in space 16, when partition 14 is formed of a polymer selected from the group consisting of a semipermeable and impermeable polymer, and when wall 12a is made of a semipermeable polymer include organic and inorganic compounds or solutes that exhibit an osmotic pressure gradient across semipermeable wall 12a against fluid in the environment, or across a semipermeable partition 14 against fluid in agent space 15. Osmotically effective compounds useful for this purpose include magnesium sulfate, magnesium chloride, sodium chloride, lithium chloride, potassium sulfate, sodium carbonate, potassium acid phosphate, mannitol, urea, sucrose, and the like. The osmotically effective compounds are also known as osmagents and they are disclosed in U.S. Patent Nos. 3,854,770 and 4,077,407.

The expressions "active agent" and "beneficial agent" as used herein broadly include any compound, composition of matter, or mixture thereof, that can be delivered from dispenser 10 to produce a beneficial and useful result. The active agents include air purifiers, algicides, antioxidants, biocides, catalysts, chemical reactants, cosmetics, contraceptives, drugs, disinfectants, food supplements, fermentation agents, fertility inhibitors, fertility promoters, fungicides, germicides, herbicides, insecticides, micro-organism attenuators, pheremones, pro-drugs, plant growth inhibitors, pesticides, preservatives, rodenticides, sex sterilants, slimicides, vitamins and other agents that benefit the environment of use and mankind.

Representative of drugs that can be delivered by dispenser 10 include tranquilizers such as reserpine, thropropazate, perphenazine and chloropromazine; psychic energizers such as amitriplyline, imipramine and methylphenidate; analgesics-antipyretics such as aspirin, phenacetin and salicylamide indomethacin diclofenac; anti-inflammatories such as hydrocortisone, dexamethazone, prednisolone, and phenylbutazone; decongestants such as phenylephrine and pseudoephedrine antibiotics such as erythromycin, tetracycline, minocyline, etc. cardiovascular drugs such as quinidire; and other agents.

Representative of drugs can can be dispensed in the vagina from a system sized, shaped and adapted for easy insertion and comfortable retention in the vagina include allantorn, aminoacridine hydrochloride, benzocaine, benzalkonium chloride, candicidin, dienestrol., dibucaine, ephedrine sulfate, furazolidone, gentain violet, hydrocortisone, methylbenzethium chloride, phenylmercuric acetate, providone-iodine, sulfanilamide, sulfisoxazole, tetracaine, undecylenate, and the like. See *Techniques of Medication*, by Eric W. Martin, pages 106 to 107, 1969, published by J. B, Lippincott Company, Philadelphia.

Representative of drugs that can be dispensed in the ano-rectal environment from a system shaped, sized and adapted for easy insertion and comfortable retention therein include acetarsol, adrenaline with benzocaine, aminophylline, aminophylline with phenobarbitol sodium, ampicillin, aspirin, astroscopolamine, belladonna, benzocaine, bisacodyl, bismuth subgallate, caffeine, ergotamine tartrate, chloralhydrate, chlorpromazine, cinchocaine, cyclomethycaine sulfate, dimenhydrinate, hydrocortizone, ichthammol, isoprenaline, metronidazole, morphine, oxymorphine hydrodiamine, thiethylperzaine meleate, and the like. See *Martindale The Extra Pharmacopolia*, Edited by Ainley Wade, General Index, page 2056, 1977, published by the Pharmaceutical Press, London; and, *National Drug Code Directory*, 1972, published by Public Health Service, U.S. Department of Health, Education and Welfare, Washington, D.C.

The drug present in dispenser 10 can be in various forms, such as uncharged molecules, molecular complexes, pro-drug, pharmacological acceptable salts such as hydrochlorides, hydrobromides, sulfate, laurylate, palmitate, phosphate, nitrate, borate, acetate, maleate, tartrate, oleates, and salicylate. For acidic drugs, salts of metals, amines, or organic cations, for example, quaternary ammonium salts can be used. Derivatives of drugs such as esters, ethers and amides, which have solubility characteristics suitable for use herein can be used. The agent or drug can be in the compartment as a suspension, dispersion, paste, cream, particle, granule, emulsion, solution, powder, and the like.

The amount of agent in dispenser 10 is preferably initially in excess of the amount that can be dissolved in fluid that enters the agent housing space. Under this physical state, when agent 17 is in excess, dispenser 10 will diffusingly operate to give a substantially constant rate of release over time, then member 18 activates and the combined action of member 18 and dispenser 10 operating as a unit system producing a substantially constant rate of release over a prolonged period of time. The length of time agent is released can also be varied by having different amounts of agent in dispenser 10 to form saturated solutions containing saturated concentrations of agent for delivery from the dispenser 10. Generally, dispenser 10 can house from 100 mg to 4 g or more, with individual devices containing for example 100 mg, 125 mg, 250 mg, or 500 mg.

The systems of the invention are manufactured by standard techniques. For example, in one embodiment a swellable polymer or a compressed amount of an osmotic solute are independently coated on one surface with a partition forming polymer, and then a compressed amount of agent, having a shape that corresponds to the shape of the polymer or solute. Next, a microporous wall forming the system can be applied by molding, spraying or dipping the system intermediate into a wall forming material to completely surround the intermediate and yield the system. In another embodiment, a microporous wall can be partly cast in a preshaped mold to the desired dimension defining the wall that surrounds an internal space, the space partly filled with a mass of agent, followed by a layer of a partition and then a mass of a driving force. The remainder of the wall abutting the driving force, is formed from a microporous or semipermeable polymer for closing the device. Walls forming the system also can be joined by various joining techniques, such as high frequency electronic sealing that provides clean edges and firmly formed walls. Another, and presently preferred technique that can be used is the air suspension procedure. Air suspension procedures are described in U.S. Patent No. 2,799,241; in *J. Am. Pharm. Assoc.,*

Vol. 49, pages 82 to 84, 1960. Other wall forming techniques include pan coating, in which the materials are deposited by successive tumbling and spraying of the polymer solution on the agent and the driving member tumbling in a rotating pan. Other standard manufacturing procedures are described in *Modern Plastic Encyclopedia*, Vol. 46, pages 62 to 70, 1069; and in *Pharmaceutical Sciences*, by Remington, 14th Ed., pages 1626 to 1678, 1970, published by Mack Publishing Company, Easton, Penna.

Exemplary solvents suitable for manufacturing the wall, or the partition include inert inorganic and organic solvents that do not adversely harm the wall forming materials, the partition forming materials, and the final device. The solvents broadly include members selected from the group consisting of aqueous solvents, and organic solvents, such as alcohols, ketones, esters, ethers, aliphatic hydrocarbons, halogenated solvents, cycloaliphatics, aromatics, heterocyclic solvents and mixtures thereof. Typical solvents include acetone, di-acetone alcohol, methanol, ethanol, isopropyl alcohol, butyl alcohol, methyl acetate, ethyl acetate, isopropyl acetate, n-butyl acetate, methyl isobutyl ketone, methyl ether, ethylene glycol monoethyl acetate, methylene dichloride, ethylene dichloride, propylene dichloride, carbon tetrachloride, isopropyl ether, cyclohexane cyclo-octane, benzene, toluene, naphtha, 1,4-dioxane, tetrahydrofuran, diglyme, water, and mixtures thereof such as acetone and water, acetone and methanol, acetone and ethyl alcohol, methylene dichloride and methanol, and ethylene dichloride and ethanol.

Examples

The following examples further illustrate the invention. The examples are not intended to limit the invention in any way.

Example 1

First, 125 mg of the osmotic solute sodium chloride is compressed to a preselected shape and coated on one of its surface with a partition forming composition comprising 70% cellulose acetate having an acetyl content of 32% mixed with 30% polyethylene glycol having a molecular weight of 400 dissolved in methylene chloride methanol, 80:20, wt:wt, until an expandable partition is formed on the solute.

Next, 235 mg of dry antiarrhythmic and antifirbrillatory p-amino-N-(2)-diethylaminoethyl)-benzamide hydrochloride having a molecular weight of 271.19 is compressed into a shape that corresponds to the shape of the solute, and placed onto the available surface of the partition. Next the intermediate dispenser comprising of the solute, partition and drug is surrounded with a microporous wall. The microporous wall is formed from a composition consisting of 65 g of cellulose acetate having an acetyl content of 32%, 41 g of the pore-former sorgitol, 11.7 g of polyethylene glycol 400, and a wall forming solvent consisting 1900 ml of acetone and 375 ml of water. The wall is formed by air tumbling until a 7 mil thick microporous wall is formed on the system to produce a system manufactured as an oral dispenser.

Example 2

A therapeutic dispenser is manufactured in the form of an oral dispenser for delivering procainamide hydrochloride to the gastrointestinal tract of a warm-blooded animal as follows: first, 200 mg of lightly cross-linked, water swellable, water insoluble polyvinyl alcohol is coated on one surface with a layer of partition forming composition consisting of 70% cellulose acetate having an acetyl content of 32% mixed with 30% polyethylene glycol having a molecular weight of 400 and dissolved in methylene chloride: methanol, 80:20, wt:wt, until a semipermeable partition is formed. Next, 235 mg of procainamide hydrochloride having a molecular weight of 271.79 is pressed onto the partition in the form of a solid mass having a shape corresponding to the shape of the polyvinyl alcohol. Then the just-formed drug-polymer intermediate dispenser is surrounded on all surfaces, except the surface of the polymer distant from the partition, with a wall of micro-porous polymeric polypropylene having a void volume of 0.565 to 0.075 $cm^3$/gm, a density of 0.60 to 0.85 $gm/cm^3$, and a pore size of 150 to 5000 angstroms, as disclosed in U.S. Patent No. 3,426,754. Finally, the exposed surface of the polyvinyl alcohol polymer is coated with a wall of cellulose acetate having an acetyl content of 38.3% to yield the dispenser.

**Claim**

A dispenser for the controlled delivery of an active agent into a fluid environment, the dispenser comprising an exterior wall surrounding and forming an internal compartment, a flexible partition in the compartment separating the compartment into a first space and a second space, an active agent in the first space and a swellable lightly cross-linked polymer or an osmotically effective solute in the second compartment, the dispenser characterized in that (a) the exterior wall is formed of a microporous polymer when the second space contains a swellable cross-linked polymer, or (b) the exterior wall is formed of a microporous and a semipermeable polymer with the semipermeable polymer surrounding a part of the second space containing a swellable cross-linked polymer or an osmotically effective solute, and in that the external wall totally encloses the internal compartment, and the microporous polymer in (a) or (b) permits the passage of active agent from the dispenser to the environment.

**Revendication**

Distributeur pour la libération contrôlée d'un agent actif dans un environnement fluide, le distributeur comprenant une paroi extérieure entourant et formant un compartiment intérieur, une cloison de séparation souple dans le compartiment séparant le compartiment en un premier espace et une second espace, un agent actif dans le premier espace et un polymère légèrement réticulé gonflable ou un soluté osmotiquement efficace dans le second compartiment, ce distributeur étant caractérisé en ce que (a) la paroi extérieure est formée d'un polymère microporeux lorsque le second espace contient un polymère réticulé gonflable, ou (b) la paroi extérieure est formée d'un polymère microporeux et d'un polymère semi-perméable, le polymère semi-perméable entourant une partie du second espace contenant un polymère réticulé gonflable ou un soluté osmotiquement efficace, et en ce que la paroi extérieure enveloppe totalement le compartiment intérieure, et le polymère microporeux dans (a) ou (b) permet le passage d'agent actif du distributeur à l'environnement.

**Patentanspruch**

Abgabevorrichtung zur geregelten Freisetzung eines Wirkstoffs in eine flüssige Umgebung, wobei die Abgabevorrichtung eine äußere Wand, die eine innere Kammer umgibt und bildet, eine flexible Trennwand in der Kammer, die die Kammer in einen ersten und einen zweiten Raum trennt, einen Wirkstoff in dem ersten Raum und ein quellfähiges leicht vernetztes Polymer oder einen osmotisch wirksamen löslichen Stoff in der zweiten Kammer umgibt, dadurch gekennzeichnet, daß (a) die äußere Wand aus einem mikroporösen Polymer besteht, wenn der zweite Raum ein quellfähiges vernetztes Polymer enthält oder (b) die äußere Wand aus einem mikroporösen und einem semipermeablen Polymer besteht, wobei das semipermeable Polymer einen Teil des zweiten Raumes umgibt, der ein quellfähiges vernetztes Polymer oder einen osmotisch wirksamen löslichen Stoff enthält und daß die äußere Wand die innere Kammer vollständig umschließt und das mikroporöse Polymer in (a) oder (b) den Durchgang des Wirkstoffs aus der Abgabevorrichtung in die Umgebung erlaubt.

FIG. 1

10
11
12

FIG.2a

10
15
17
18
11
12
13
16
14

FIG. 2b

10
15
17
16
14
11
12
13
18
12a

FIG. 3

19
13
15
10
17
11
14
12
13
16
18
20
12a
21